# EUROPEAN PATENT APPLICATION

(11) **EP 0 819 940 A2**
(43) Date of publication of application: **21.01.1998**
(21) Application number: 97305179.0
(22) Date of filing: 14.07.1997
(51) Int. Cl.: G01N 33/497, G01N 31/22

(54) **Method of detecting halitosis and detector for use in the method**

(30) Priority: 15.07.1996 ZA 6000296
(71) Applicant: Marx, Ivan, Pretoria 0001 (ZA)
(72) Inventor: Marx, Ivan, Pretoria 0001 (ZA)
(74) Representative: Perkins, Sarah

(57) **Abstract**

The invention provides a gas detector for detecting halitosis. The gas detector comprises a translucent container having a breath inlet with a mouthpiece attached to and extending therefrom. The container contains a detection agent in its interior which is visible through the container to allow visual detection of reaction of the detection agent with at least one undesirable gaseous compound in a person's breath in use. The reaction results in a visible colour change of the detection agent to indicate whether or not a person has halitosis. The container also includes a non-return inlet valve provided between the mouthpiece and the interior of the container.

## Description

THIS INVENTION relates to detection of halitosis or undesirable compounds in a person's breath. More particularly, this invention relates to a gas detector for detecting halitosis, and to a method of detecting halitosis using the gas detector.

According to one aspect of the invention, there is provided a method of detecting halitosis, the method comprising the steps of:
bringing a person's breath into contact with a detection agent by having the person blow or breathe into a gas detector as described hereunder; and
determining visually whether or not an detectable change has taken place in the detection agent as a result of the reaction of at least one undesirable compound in the person's breath with the detection agent, the detectable change providing an indication of the presence of such compound.

The undesirable compounds in the person's breath will typically be those compounds which cause halitosis or bad breath, such as volatile sulphur-containing compounds, e.g. hydrogen sulphide (H₂S), thiols or mercaptans, alkyl sulphides such as dimethyl sulphide, or the like.

The breath will be brought in contact with or pass over or through the detection agent for a sufficient period to allow the reaction between the detection agent and one or more of the undesirable compounds, if present in sufficient or undesirable proportions in the breath, to form a detectable visible reaction product, eg by way of a colour change.

The gas detector may be provided with the detection agent in solid form, e.g. a dry sorbent substrate which has been impregnated with the detection agent. Instead, the detection agent may be provided in liquid form, e.g. water having the detection agent dissolved therein. Naturally, the amount or concentration of the detection agent impregnated into the substrate, or the concentration of the detection agent dissolved in the water, will normally be determined by routine experimentation for effective detection of the presence in the breath of undesirable amounts of commonly occurring undesirable compound(s).

According to another aspect of the invention, there is provided a gas detector for detecting halitosis, the gas detector comprising a translucent container having a breath inlet with a mouthpiece attached to and extending therefrom, and the container containing a detection agent in its interior, the detection agent being visible through the container to allow visual detection of reaction of the detection agent with at least one undesirable gaseous compound in a person's breath in use which results in a visible colour change of said detection agent. a non-return inlet valve being provided between the mouthpiece and the interior of the container.

As mentioned above, specific undesirable compounds in the person's breath which can result in a person being said to have halitosis or tad breath, will be detected. Preferably, the specific compounds to be detected are usually volatile sulphur-containing compounds such as hydrogen sulphide (H₂S), thiols or mercaptans, alkyl sulphides such as dimethyl sulphide, or the like.

The gas detector may include a sorbent substrate containing, impregnated therein, the detection agent. The sorbent substrate may be secured to the interior of the gas detector, e.g. adhesively. Accordingly, the detection agent may be impregnated in a sorbent porous substrate located in the interior of the container, the container being transparent. In principle, it is possible to impregnate the substrate with more than one detection agent, depending on the type of compound(s) to be detected. Any suitable material can be used for the substrate. The substrate may be in the form of paper, eg a strip of paper, or in the form of cotton wool. The sorbent substrate may be divided into a plurality of portions, each portion adhesively secured to the interior of the container.

Any suitable shape of container may be used with an inlet in the form of an opening or aperture being provided in the container as mentioned above to allow introduction of breath into the container in use. As mentioned above, the inlet aperture in the container may be provided with a one-way- or non-return inlet valve, the valve being arranged only to allow introduction of the breath into the interior of the container. Preferably, the container is in the form of an elongated conduit or tube, one end of the tube being a closed end and the other end being an open end to provide the inlet aperture into the tube.

in one embodiment of the invention, the container may comprise a tube having an open end defining the breath inlet and a closed end, the non-return inlet valve being provided at the open end of the tube with the mouthpiece being integral with the open end.

In another embodiment, the gas detector may contain a detection solution, e.g. an aqueous solution, with the detection agent being the solute dissolved in the solvent of the solution. Naturally, more than one detection agent can be dissolved in the solvent. The container may be transparent, the detection agent being in the form of a solute dissolved in a solvent, the mouthpiece being integral with the breath inlet and the solute and solvent forming the detection solution located in the container.

In a further embodiment of the invention, the container may comprise a tube containing the detection solution, the gas detector comprising a conduit in gaseous flow communication with the inlet and with the interior of the tube via at least one said non-return valve to allow one-way flow of breath into the detection solution in the interior of the tube in use. The interior of the tube may be divided by partitions into a plurality of compartments, each compartment being provided with a said non-return inlet valve. The container may be provided with at least one outlet to permit breath to escape from the container.

When the gaseous compound is a more or less volatile sulphur-containing compound, the detection agent may be lead acetate [Pb(CH₃COO)₂] together with at least one other agent which turns black as a result of reacting with the sulphur-containing compound in a cascade reaction. Naturally, the type of detection agent will be selected depending on the type of gaseous constituent(s) to be detected. The detection agent may be selected from the group consisting of Pb(CH₃COO)₂, PbS, CuS, HgS, Ag₂S, Bi₂S₃, Bi₂S₄, Bi₂S₅, Bi₂S₆, Bi₂S₇, and mixtures thereof, or any other suitable detection agent. A mixture of detection agents may be used which act together, e.g. a cascade reaction, to provide a visible colour change when exposed to at least one undesirable compound in a person's breath.

The container may be of glass, transparent plastics material or any other suitable transparent or translucent material. Naturally, the detection agent must be visible to a user of the gas detector so that the user can determine whether or not a visually detectable change, e.g. a colour change, has taken place.

The gas detector may be contained in an airtight package. A plurality of said gas detectors may be packaged in individual chambers in a ccmmon package to form a detection pack, or they may be contained in separate packages. If necessary, the detection agent may be shielded from external or ambient gases, to reduce or prevent ambient gas from reacting with the detection agent prior to use thereof. In other word, the gas detector may be enclosed in a sealed, airtight package to reduce or prevent any adverse effects which environmental or ambient gases may have on the detection agent.

In some cases, the detection agent can undergo degradation with time. The gas detector may thus be provided with some form of indication as to the shelf-life thereof, e.g. such as an expiry date printed on the container cf the gas detector or the package. Accordingly, the gas detector may comprise a shelf-life marking. In another embodiment of the invention, the gas detector may include a shelf-life expiry agent which is selected visibly to change colour with time to indicate when the shelf-life of the detection agent in the gas detector has expired.

The gas detector may include a control agent which changes colour when exposed to a person's breath irrespective of the presence of halitosis to indicate whether or not the gas detector has already been used.

The gas detector may be provided with a set of instructions for instructing a user thereof how to use the gas detector. The set of instructions may also include a colour reference chart for comparison with the detection agent to determine whether or not a person has halitosis.

The gas detector may contain a filter means or absorption means between the breath inlet and the detection agent for removing certain compounds, which compounds could influence or interfere with the reaction of the detection agent, from a person's breath, e.g. carbon dioxide and/or for water vapour, before it comes into contact with the detection agent. In one embodiment of the invention, the absorption means may comprise soda lime for at least partial removal of carbon dioxide and water from a person's breath when it passes through the soda lime before coming into contact with the detection agent.

In a further embodiment of the invention, the container may comprise a tube having an open end defining the breath inlet with the mouth piece being integral with the open end, a non-return inlet valve being provided at or adjacent the open end of the tube, the tube including a compartment containing absorption means therein and having an inlet aperture leading into the compartment and an outlet aperture leading out of the compartment, at least one sorbent substrate impregnated with a detection agent provided downstream of the compartment in the interior of the tube, and the tube having an outlet to permit breath to escape from the tube once it has passed through the non-return valve, the compartment and come into contact with the detection agent.

The gas detector may include an indicator agent provided in the interior of the container for indicating by means of a visible colour change whether or not the detection agent has been exposed to external or ambient gases prior to use of the gas detector. In other words, the indicator agent is selected to change colour as a result of coming into contact with environmental or ambient gases.

The invention also extends to a kit for detecting halitosis, the kit comprising at least one gas detector as herein described and a set of instructions.

The kit may include a colour reference chart for comparison with the detection agent once the detection agent has been exposed to a person's breath to determine whether or not the person has halitosis.

The invention will now be described, by way of non-limiting example, with reference to the accompanying diagrammatic drawings, in which:
Figure 1 shows a schematic side view of an embodiment of a gas detector according to the invention;
Figure 2 shows a schematic side view of another embodiment of a gas detector according to the invention; and
Figure 3 shows a schematic side view of a further embodiment of a gas detector according to the invention.

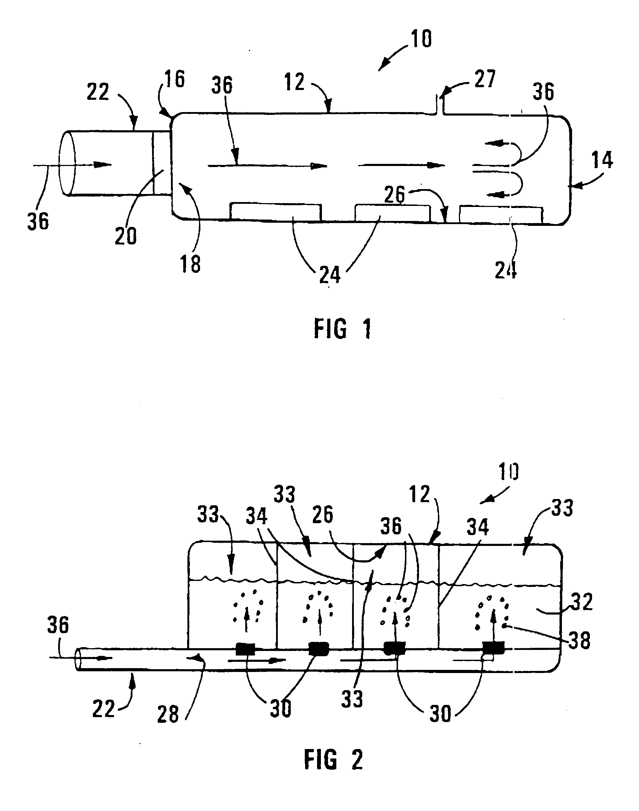

In Figure 1, reference numeral 10 generally indicates a gas detector according to the invention. The detector 10 comprises a translucent disposable plastics tube 12 having a closed end 14 and an open end 16, the open end 16 defining a breath inlet 18 leading into the tube 12. A one-way or non-return inlet valve 20 is provided in the breath inlet 18 and a mouthpiece 22 is connected to and extends from the valve 20. Three strips of paper 24, impregnated with a detection agent, e.g. a metal salt in combination with one or more other agents which undergo a cascade reaction when exposed to halitosis, are adhesively secured to an inner surface 26 of a wall of the tube 12 in spaced apart fashion as shown. The tube 12 has a gas escape bleed outlet at 27.

Referring to Figure 2, the same reference numerals are used to indicate the same parts of Figure 1 unless otherwise specified. The main difference between Figure 1 and Figure 2 is that in Figure 2 the mouthpiece 22 is integral with a thin conduit or straw 28. The straw 28 is connected in flow communication with the interior of the tube 12 via one-way valves 30 as illustrated. The tube 12 is of transparent plastics material. An aqueous detection solution 32 with a detection agent dissolved therein is contained in the interior of the tube 12, the tube interior being divided lengthwise into a plurality (four) compartments 33 by three partitions 34. There are no outlets from these compartments 33.

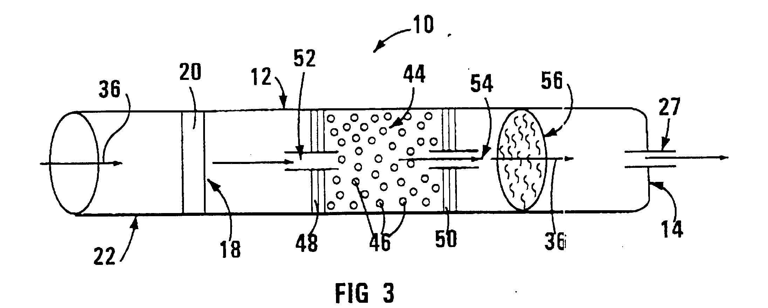

Referring to Figure 3, the same reference numerals are used to indicate the same parts of Figures 1 and 2 unless otherwise specified. In Figure 3, the mouthpiece 22 is integral with the breath inlet 18 and the non-return inlet valve 20 is provided in the breath inlet 18. The interior of the tube 12 is provided with a compartment 40 which is filled with soda lime particles 46. The compartment 44 is defined by two plugs or partitions 48, 50 which serve to retain the soda lime particles 46 in the compartment 44. The partition 48 has an inlet aperture 52 and the partition 50 has an outlet aperture 54. A wad of cotton wool 56, impregnated with a detection agent, is provided in the interior of the tube 12 downstream of the compartment 44 as shown. The tube 12 has a gas outlet 27 provided in its end 14.

Optionally, the gas detector 10 of Figures 1 - 3 includes an indicator agent provided in the interior of the container for indicating by means of a visible colour change whether or not the detection agent has been exposed to external or ambient gases prior to use of the gas detector. The gas detector 10 also optionally includes a shelf-life expiry agent, which agent is selected visibly to change colour with time to indicate when the shelf-life of the detection agent in the gas detector 10 has expired. Furthermore, the gas detector 10 optionally includes a control agent which visibly changes colour when exposed to a person's breath irrespective of the presence of halitosis to indicate whether or not the gas detector has already been used.

The gas detectors 10 of Figures 1 - 3 are packed for distribution and storage in sealed airtight packages (not shown). In one embodiment, a plurality of the gas detectors 10 are packaged in individual chambers in a common package (not shown) to form a detection pack.

The invention also provides a kit for detecting halitosis, the kit comprising one or more gas detectors 10 together with a set of instructions for instructing a user thereof how to use the gas detector 10. Optionally, the set of instructions includes a colour reference chart for comparison with the detection agent once it has been exposed to a person's breath to determine whether or not the person has halitosis. The instructions may also specify what corrective action needs to be taken if the gas detector indicates that the person has halitosis.

In use, referring specifically to Figure 1, a person who wishes to test whether or not he has bad breath will tear open the package containing the detector 10 and remove the detector 10 from the package. The person will then place his lips over or against the mouthpiece 22 and blow into and through the detector 10 in the director of arrows 36. Escape of his breath from the tube 12 is permitted by the outlet 27. If the person has bad breath, his breath will typically contain gaseous volatile sulphur-containing compounds such as hydrogen sulphide (H₂S), thiols, mercaptans or the like. The person then waits a sufficient time, usually a few seconds, to see whether or not such compounds in his breath cause a colour change of the detection agent. If his breath contains such compounds, they will react with the detection agent and in sufficient amounts will cause the detection agent visibly to change colour. The person can see that the detection agent has changed colour and therefore knows that he has bad breath. The person can then take whatever steps are necessary as are set out in the instructions to alleviate this problem.

Referring now specifically to Figure 2, the same procedure as mentioned above for the tube 12 of Figure 1 is carried out except that the person places his lips around the mouthpiece 22 of the straw 28 and blows into the straw 28 in the direction of the arrows 36. The person's breath is introduced into the solution 32 through the one-way valves 30, which valves 30 inhibit or prevent the solution 32 escaping from the tube 12. The person's breath travels through and comes into contact with the solution 32 in the form of bubbles 36. The person then waits a very short period, e.g.. a few seconds, to see whether or not a visible detectable colour change of the solution 32 takes place. As there is no outlet from the tube 12, there will be a pressure increase in its compartments 33 as the user blows into the straw 28.

Turning to Figure 3, the same procedure as mentioned above for Figures 1 and 2 is carried out. The person's breath passes through the mouthpiece 22, through the non-return inlet valve 20 and passes into the compartment 44 through the aperture 52. In the compartment 44, the soda lime particles 46 absorb at least some of the carbon dioxide and water vapour present in the person's breath whereafter the person's breath passes through the outlet aperture 54. The person's breath then passes through the cotton wool 56 where it comes into contact with the detection agent impregnated therein before it passes out of the tube through the outlet 27. After a few seconds, the detection agent will change colour if there are any undesirable compounds such a hydrogen sulphide, thiols, mercaptans or the like in the person's breath. The person then compares the colour of the detection agent to the colour reference chart provided with the gas detector 10 to determine whether or not he has halitosis. If the colour of the detection agent indicates that the person has halitosis, the person then reads the instructions to determine what corrective action needs to be taken to alleviate this problem.

The invention will now be described, by way of non-limiting example, with reference to tests carried out for various detection agents as listed in the Table hereunder.

**TABLE -**

| Sensitivity of Metal Salts to Hydrogen Sulphide (H₂S) | | | | | | | | |
|---|---|---|---|---|---|---|---|---|
| Tests were carried out on various metal salts to determine their sensitivity to or reaction with hydrogen sulphide. The results of the tests are set out in the Table below. | | | | | | | | |

| METAL SALT | SOLUBILITY | CONCENTRATION | FREE [S⁻²] | pH | [H⁺] | [H₂S] | X_{g} | Px |
|---|---|---|---|---|---|---|---|---|
| PbS | 8.81E-29 | 1.00E-03 | 8.81E-26 | 4 | 0.0001 | 7.87E-12 | 1.42E-13 | 7.72E-11 |
| PbS | 8.81E-29 | 1.00E-03 | 8.81E-26 | 6 | 1E-06 | 7.87E-16 | 1.42E-17 | 7.72E-15 |
| PbS | 8.81E-29 | 1.00E-03 | 8.81E-26 | 7 | 1E-07 | 7.87E-18 | 1.42E-19 | 7.72E-17 |
| PbS | 8.81E-29 | 1.00E-03 | 8.81E-26 | 8 | 1E-08 | 7.87E-20 | 1.42E-21 | 7.72E-19 |
| PbS | 8.81E-29 | 1.00E-03 | 8.81E-26 | 10 | 1E-10 | 7.87E-24 | 1.42E-25 | 7.72E-23 |
| CuS | 1.28E-36 | 1.00E-03 | 1.28E-33 | 4 | 0.0001 | 1.14E-19 | 2.06E-21 | 1.12E-18 |
| CuS | 1.28E-36 | 1.00E-03 | 1.28E-33 | 6 | 1E-06 | 1.14E-23 | 2.06E-25 | 1.12E-22 |
| CuS | 1.28E-36 | 1.00E-03 | 1.28E-33 | 7 | 1E-07 | 1.14E-25 | 2.06E-27 | 1.12E-24 |
| CuS | 1.28E-36 | 1.00E-03 | 1.28E-33 | 8 | 1E-08 | 1.14E-27 | 2.06E-29 | 1.12E-26 |
| CuS | 1.28E-36 | 1.00E-03 | 1.28E-33 | 10 | 1E-10 | 1.14E-31 | 2.06E-33 | 1.12E-30 |
| HgS | 6.38E-53 | 1.00E-03 | 6.38E-50 | 4 | 0.0001 | 5.7E-36 | 1.03E-37 | 5.59E-35 |
| HgS | 6.38E-53 | 1.00E-03 | 6.38E-50 | 6 | 1E-06 | 5.7E-40 | 1.03E-41 | 5.59E-39 |
| HgS | 6.38E-53 | 1.00E-03 | 6.38E-50 | 7 | 1E-07 | 5.7E-42 | 1.03E-43 | 5.59E-41 |
| HgS | 6.38E-53 | 1.00E-03 | 6.38E-50 | 8 | 1E-08 | 5.7E-44 | 1.03E-45 | 5.59E-43 |
| HgS | 6.38E-53 | 1.00E-03 | 6.38E-50 | 10 | 1E-10 | 5.7E-48 | 1.03E-49 | 5.59E-47 |
| Ag₂S | 6.62E-50 | 1.00E-03 | 6.62E-47 | 4 | 0.0001 | 5.91E-33 | 1.06E-34 | 5.8E-32 |
| Ag₂S | 6.62E-50 | 1.00E-03 | 6.62E-47 | 6 | 1E-06 | 5.91E-37 | 1.06E-38 | 5.8E-36 |
| Ag₂S | 6.62E-50 | 1.00E-03 | 6.62E-47 | 7 | 1E-07 | 5.91E-39 | 1.06E-40 | 5.8E-38 |
| Ag₂S | 6.62E-50 | 1.00E-03 | 6.62E-47 | 8 | 1E-08 | 5.91E-41 | 1.06E-42 | 5.8E-40 |
| Ag₂S | 6.62E-50 | 1.00E-03 | 6.62E-47 | 10 | 1E-10 | 5.91E-45 | 1.06E-46 | 5.8E-44 |
| Bi₂S₃ | 1.80E-99 | 1.00E-03 | 1.8E-96 | 4 | 0.0001 | 1.61E-82 | 2.89E-84 | 1.58E-81 |
| Bi₂S₄ | 1.80E-99 | 1.00E-03 | 1.8E-96 | 6 | 1E-06 | 1.61E-86 | 2.89E-88 | 1.58E-85 |
| Bi₂S₅ | 1.80E-99 | 1.00E-03 | 1.8E-96 | 7 | 1E-07 | 1.61E-88 | 1.89E-90 | 1.58E-87 |
| Bi₂S₆ | 1.80E-99 | 1.00E-03 | 1.8E-96 | 8 | 1E-08 | 1.61E-90 | 1.89E-92 | 1.58E-89 |
| Bi₂S₇ | 1.80E-99 | 1.00E-03 | 1.8E-96 | 10 | 1E-10 | 1.61E-94 | 1.89E-96 | 1.58E-93 |

The results shown in the Table indicate which metal salts are the most reactive or sensitive to hydrogen sulphide and are most suitable for use as a detection agent in the gas detector in accordance with the invention.

The preferred detection limits of the undesirable compounds by the gas detector in accordance with the invention are 100 - 300 parts per billion.

| For comparison: | |
|---|---|
| Human detection threshold | 2.10E10 |
| Human recognition or identification threshold | 4.70E-10 |
| Strong Smell | 5.00E07 |
| Headache, irritation | 1.00E05 |

Advantages of the present invention include ease of use of the gas detector to determine whether or not one has bad breath and the relatively rapid rate in which a person can carry out a test for bad breath, usually a few seconds. Furthermore, gas detectors in accordance with the invention can be manufactured relatively inexpensively; and the amount of detection agent used can be selected by routine experimentation to give a detectable colour change within a minute, eg in several seconds, in response to undesirable amounts of undesirable compounds in a person's breath.

## Claims

1. A gas detector for detecting halitosis, the gas detector characterized in that it comprises a translucent container having a breath inlet with a mouthpiece attached to and extending therefrom, and the container containing a detection agent in its interior, the detection agent being visible through the container to allow visual detection of reaction of the detection agent with at least one undesirable gaseous compound in a person's breath in use which results in a visible colour change of said detection agent, a non-return inlet valve being provided between the mouthpiece and the interior of the container.

2. A gas detector as claimed in claim 1, characterized in that the detection agent is impregnated in a sorbent porous substrate located in the interior of the container, the container being transparent.

3. A gas detector as claimed in claim 2, characterized in that the sorbent substrate is divided into a plurality of portions, each portion adhesively secured to the interior of the container.

4. A gas detector as claimed in any one of claims 1 - 3, characterized in that the container comprises a tube having an open end defining the breath inlet and a closed end, the non-return inlet valve being provided at the open end of the tube with the mouthpiece being integral with the open end.

5. A gas detector as claimed in claim 1, characterized in that the container is transparent, the detection agent being in the form of a solute dissolved in a solvent, the mouthpiece being integral with the breath inlet and the solute and solvent forming a detection solution located in the container.

6. A gas detector as claimed in claim 5, characterized in that the container comprises a tube containing the detection solution, the gas detector comprising a conduit in gaseous flow communication with the inlet and with the interior of the tube via at least one said non-return inlet valve.

7. A gas detector as claimed in claim 6, characterized in that the interior of the tube is divided by partitions into a plurality of compartments, each compartment being provided with a said non-return inlet valve.

8. A gas detector as claimed in any one of the preceding claims, characterized in that the container is provided with at least one outlet to permit breath to escape from the container.

9. A gas detector as claimed in any one of the preceding claims, characterized in that the detection agent is selected from the group consisting of Pb(CH₃COO)₂, PbS, CuS, HgS, Ag₂S, Bi₂S₃, Bi₂S₄, Bi₂S₅, Bi₂S₆, Bi₂S₇, and mixtures thereof.

10. A gas detector as claimed in any one of the preceding claims, characterized in that it is contained in an airtight package.

11. A gas detector as claimed in any one of the preceding claims, characterized in that a plurality of said gas detectors are packaged in individual chambers in a common package to form a detection pack.

12. A gas detector as claimed in claim 10 or claim 11, characterized in that it comprises a shelf-life marking.

13. A kit for detecting halitosis, the kit characterized in that it comprises at least one gas detector as claimed in any one of claims 1 - 12 or 15 and a set of instructions.

14. A kit as claimed in claim 14, characterized in that it includes a colour reference chart.

15. A gas detector as claimed in claim 1, substantially as herein described and illustrated.

16. A kit as claimed in claim 13, substantially as herein described.
